(19) Europäisches Patentamt
European Patent Office
Office européen des brevets

(11) **EP 3 801 276 B1**

(12) **EUROPÄISCHE PATENTSCHRIFT**

(45) Veröffentlichungstag und Bekanntmachung des Hinweises auf die Patenterteilung:
**06.07.2022   Patentblatt 2022/27**

(21) Anmeldenummer: **19729728.6**

(22) Anmeldetag: **06.06.2019**

(51) Internationale Patentklassifikation (IPC):
**A61B 8/06** (2006.01)    **A61B 8/12** (2006.01)
**A61B 8/08** (2006.01)    **A61B 5/026** (2006.01)
**A61B 5/00** (2006.01)    **A61M 60/135** (2021.01)
**A61M 60/50** (2021.01)

(52) Gemeinsame Patentklassifikation (CPC):
**A61B 8/06; A61B 5/026; A61B 5/686; A61B 8/12; A61B 8/488; A61M 60/135; A61M 60/50;**
A61B 8/0883; A61B 8/0891; A61M 2205/04; A61M 2205/3334; A61M 2210/125

(86) Internationale Anmeldenummer:
**PCT/EP2019/064804**

(87) Internationale Veröffentlichungsnummer:
**WO 2019/234164 (12.12.2019 Gazette 2019/50)**

(54) **VERFAHREN ZUR BESTIMMUNG EINER STRÖMUNGSGESCHWINDIGKEIT EINES DURCH EIN IMPLANTIERTES, VASKULÄRES UNTERSTÜTZUNGSSYSTEM STRÖMENDEN FLUIDS UND IMPLANTIERBARES VASKULÄRES UNTERSTÜTZUNGSSYSTEM**

METHOD FOR DETERMINING A FLOW RATE OF A FLUID FLOWING THROUGH AN IMPLANTED VASCULAR SUPPORT SYSTEM, AND IMPLANTABLE VASCULAR SUPPORT SYSTEM

PROCÉDÉ POUR LA DÉTERMINATION D'UNE VITESSE D'ÉCOULEMENT D'UN FLUIDE S'ÉCOULANT DANS UN SYSTÈME DE SUPPORT VASCULAIRE IMPLANTÉ ET SYSTÈME DE SUPPORT VASCULAIRE IMPLANTABLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **06.06.2018   DE 102018208929**

(43) Veröffentlichungstag der Anmeldung:
**14.04.2021   Patentblatt 2021/15**

(73) Patentinhaber: **Kardion GmbH**
**70376 Stuttgart (DE)**

(72) Erfinder:
• **SCHLEBUSCH, Thomas Alexander**
**71272 Renningen (DE)**
• **SCHMID, Tobias**
**70197 Stuttgart (DE)**

(74) Vertreter: **Gauss, Nikolai et al**
**Pfiz/Gauss Patentanwälte PartmbB**
**Tübingerstraße 26**
**70178 Stuttgart (DE)**

(56) Entgegenhaltungen:
**US-A1- 2008 133 006    US-A1- 2008 210 016**

• **UDESEN J ET AL: "A simple method to reduce aliasing artifacts in color flow mode imaging", ULTRASONICS SYMPOSIUM, 2005 IEEE ROTTERDAM, THE NETHERLANDS 18-21 SEPT. 2005, PISCATAWAY, NJ, USA,IEEE, Bd. 2, 18. September 2005 (2005-09-18), Seiten 1352-1355, XP010899095, DOI: 10.1109/ULTSYM.2005.1603104 ISBN: 978-0-7803-9382-0**

- MCCORMICK W S ET AL: "Resolution of a 2/spl pi/ ambiguity problem in multiple frequency spectral estimation", IEEE TRANSACTIONS ON AEROSPACE AND ELECTRONIC SYSTEMS, IEEE SERVICE CENTER, PISCATAWAY, NJ, US, Bd. 31, Nr. 1, 1. Januar 1995 (1995-01-01) , Seiten 2-8, XP011279629, ISSN: 0018-9251, DOI: 10.1109/7.366287
- SYRMOS V ET AL: "A generalized Bezout equation in output feedback design", PROCEEDINGS OF THE CONFERENCE ON DECISION AND CONTROL. TUCSON, DEC. 16 - 18, 1992; [PROCEEDINGS OF THE CONFERENCE ON DECISION AND CONTROL], NEW YORK, IEEE, US, Bd. CONF. 31, 16. Dezember 1992 (1992-12-16), Seiten 3590-3594, XP010107496, ISBN: 978-0-7803-0872-5
- HE KONG ET AL: "A Stein equation approach for solutions to the Diophantine equations", CONTROL AND DECISION CONFERENCE (CCDC), 2010 CHINESE, IEEE, PISCATAWAY, NJ, USA, 26. Mai 2010 (2010-05-26), Seiten 3024-3028, XP031699887, ISBN: 978-1-4244-5181-4

**Beschreibung**

Die Erfindung betrifft ein Verfahren zur Bestimmung einer Strömungsgeschwindigkeit eines durch ein implantiertes, vaskuläres Unterstützungssystem strömenden Fluids, ein implantierbares, vaskuläres Unterstützungssystem sowie eine Verwendung von gepulsten Dopplermessungen mit unterschiedlichen Pulswiederholraten. Die Erfindung findet insbesondere Anwendung bei (voll-)implantierten Linksherz-Unterstützungssystemen (LVAD).

Es ist bekannt Ultraschall-Volumenstromsensoren in Herzunterstützungssysteme zu integrieren, um mit ihnen den sogenannten Pumpenvolumenstrom zu erfassen, der den Fluid-Volumenstrom durch das Unterstützungssystem selbst quantifiziert. Die Ultraschall-Volumenstromsensoren können dabei gepulste Dopplermessungen durchführen bzw. das gepulste Doppler-(engl.: Pulsed Wave Doppler; kurz: PWD)-Verfahren einsetzen. Dieses benötigt nur ein Ultraschall-Wandlerelement und ermöglicht eine genaue Wahl des Abstandes des Beobachtungsfensters vom Ultraschall-Element. Bei bekannten PWD-System werden Ultraschallimpulse mit einer festgelegten Pulswiederholrate (PRF) ausgesendet. Dabei muss die Pulswiederholrate die doppelte, maximal auftretende Doppler-Frequenzverschiebung übertreffen, um das Nyquist-Theorem nicht zu verletzten. Ist diese Bedingung nicht erfüllt, kommt es zu Aliasing, d. h. Doppeldeutigkeiten im erfassten Frequenzspektrum.

Aufgrund der geometrischen Gestaltung des Messaufbaus in Herzunterstützungssystemen (VAD) liegt der Messbereich bzw. das Beobachtungsfenster unter Umständen so weit vom Ultraschallwandler entfernt, dass die Signallaufzeit des Ultraschallimpulses vom Wandler zum Messbereich und zurück zum Wandler nicht vernachlässigt werden kann. Da beim PWD-Verfahren ein neuer Ultraschallimpuls (zumindest theoretisch) erst ausgesendet werden darf bzw. sollte, wenn der vorangegangene keine signifikanten Echos mehr liefert, limitiert die Signallaufzeit die maximal mögliche Pulswiederholrate. Bei den üblicherweise hohen in Herzunterstützungssystemen vorherrschenden Strömungsgeschwindigkeiten und den geometrischen Randbedingungen für die Entfernung des Beobachtungsfensters vom Ultraschallelement, kommt es in der Regel zu einer Verletzung des Nyquist-Abtasttheorems wodurch Doppeldeutigkeiten (Aliasing) im Spektrum entstehen.

Herzunterstützungssysteme mit Ultraschallsensoren, die nicht das PWD-Verfahren anwenden, sind üblicherweise mit zwei Ultraschallwandlern ausgestattet, sodass die beschriebene Laufzeitproblematik zwar auftreten kann, aber bei entsprechender Umsetzung anderweitig gelöst werden kann. Herzunterstützungssysteme mit Ultraschallsensoren, die das PWD-Verfahren anwenden sind jedoch insbesondere für mittlere bis hohe Flussgeschwindigkeiten anfällig für den beschriebenen Effekt. Stand der Technik ist derzeit die Maßgabe, die festgelegte Pulswiederholrate so zu wählen, dass Aliasing nicht auftritt.

Die US 2008/0133006 A1 offenbart eine Blutpumpe mit einem Ultraschallsensor, der in oder an einer Blutkontaktfläche der Blutpumpe angebracht ist. Der Ultraschallsensor misst die Blutgeschwindigkeit und meldet Informationen an eine Blutpumpensteuerung, wobei der Ultraschallsensor dazu bestimmt ist, die Blutgeschwindigkeit in einer mit der Blutpumpe verbundenen Einströmkanüle zu messen.

Die US 2008/0210016 A1 beschreibt die Bestimmung eines Aliasing-freien Radialgeschwindigkeitsspektrums von sich bewegender Materie mittels gepulster Ultraschallwellen.

Aufgabe der Erfindung ist es, ein verbessertes Verfahren zur Bestimmung einer Strömungsgeschwindigkeit eines durch ein implantiertes, vaskuläres Unterstützungssystem strömenden Fluids anzugeben und ein verbessertes implantierbares vaskuläres Unterstützungssystem zu schaffen, in dem die einer Strömungsgeschwindigkeit eines durch dieses strömenden Fluids bestimmt werden kann.

Insbesondere ist es eine Aufgabe der Erfindung, ein Verfahren zur Bestimmung einer Strömungsgeschwindigkeit eines Fluids und ein verbessertes implantierbares vaskuläres Unterstützungssystem zu schaffen, in dem das Bestimmen der Strömungsgeschwindigkeit eines durch dieses strömenden Fluids vorgesehen ist, bei dem mit nur einem Ultraschallwandler das Bestimmen der Strömungsgeschwindigkeit bei den in einem Herzunterstützungssystem herrschenden Strömungsgeschwindigkeiten auch bei großer Signallaufzeit eines Ultraschallimpulses von dem Ultraschallwandler zum Messbereich und zurück möglich ist.

Diese Aufgabe wird durch das in Anspruch 1 angegebene Verfahren und das implantierbare, vaskuläre Unterstützungssystem nach Anspruch 7 gelöst.

Vorteilhafte Ausführungsformen der Erfindung sind in den abhängigen Ansprüchen angegeben.

Hier vorgeschlagen wird gemäß Anspruch 1 ein Verfahren zur Bestimmung einer Strömungsgeschwindigkeit $v$ eines durch ein implantiertes, vaskuläres Unterstützungssystem strömenden Fluids, umfassend:

a) Das Durchführen einer ersten gepulsten Dopplermessung mit einer ersten Pulswiederholrate $PRF_1$ mittels eines Ultraschallsensors des Unterstützungssystems,

b) das Durchführen einer zweiten gepulsten Dopplermessung mit einer zweiten Pulswiederholrate $PRF_2 > PRF_1$ mittels des Ultraschallsensors des Unterstützungssystems, wobei sich die zweite Pulswiederholrate von der ersten Pulswiederholrate unterscheidet, und

c) das Ermitteln der Strömungsgeschwindigkeit unter Verwendung von Messergebnissen der ersten gepulsten Dopplermessung und der zweiten gepulsten Dopplermessung, indem für ganzzahliges $n_1$, $n_2$ und einer Hauptkomponente $f_1$ der ersten gepulsten Dopplermessung und einer Hauptkomponente $f_2$ der zweiten gepulsten Dopplermessung folgende lineare Diophantische Gleichung

$$n_1 \cdot PRF_1 - n_2 \cdot PRF_2 = f_1 - f_2$$

für folgende Randbedingung

$$v < \frac{a \cdot PRF_1 \cdot c_0}{2f_0}$$

unter der Annahme gelöst wird:

$$-|a| \leq n_1 \leq |a|$$

und

$$-|b| \leq n_2 \leq |b|,$$

wobei

$$a := \frac{PRF_2}{2 \cdot ggT(PRF_1, PRF_2)}$$

und

$$b := \frac{PRF_1}{2 \cdot ggT(PRF_1, PRF_2)},$$

und wobei $f_0$ die Ultraschall-Sendefrequenz des Ultraschallsensors und $c_0$ die Schallgeschwindigkeit in dem Fluid ist.

Das vaskuläre Unterstützungssystem ist bevorzugt ein kardiales Unterstützungssystem, besonders bevorzugt ein ventrikuläres Unterstützungssystem. Regelmäßig dient das Unterstützungssystem zur Unterstützung der Förderung von Blut im Blutkreislauf eines Menschen, ggf. Patienten. Das Unterstützungssystem kann zumindest teilweise in einem Blutgefäß angeordnet sein. Bei dem Blutgefäß handelt es sich beispielsweise um die Aorta, insbesondere bei einem Linksherz-Unterstützungssystem, oder um den gemeinsamen Stamm (Truncus pulmonalis) in die beiden Lungenarterien, insbesondere bei einem Rechtsherz-Unterstützungssystem. Das Unterstützungssystem ist bevorzugt am Ausgang des linken Ventrikels des Herzens bzw. der linken Herzkammer angeordnet. Besonders bevorzugt ist das Unterstützungssystem in Aortenklappenposition angeordnet.

Die hier vorgeschlagene Lösung trägt insbesondere zur Kompensation von Aliasingeffekten in einem medizinischen Pulsed Wave Doppler System bei. Das Verfahren kann zur Bestimmung einer Fluid-Strömungsgeschwindigkeit und/oder eines Fluid-Volumenstroms aus einem Ventrikel eines Herzens, insbesondere von einem (linken) Ventrikel eines Herzens hin zur Aorta im Bereich eines (voll-)implantierten, (links-)ventrikulären (Herz-)Unterstützungssystems beitragen. Bei dem Fluid handelt es sich regelmäßig um Blut. Die Strömungsgeschwindigkeit wird in einem Fluidstrom bzw. Fluid-Volumenstrom bestimmt, der durch das Unterstützungssystem, insbesondere durch eine (Einlauf-)Kanüle des Unterstützungssystems hindurch strömt. Das Verfahren ermöglicht in vorteilhafter Weise, dass die Strömungsgeschwindigkeit und/oder der Fluid-Volumenstrom des Blut-Flusses auch außerhalb des OP-Szenarios mit hoher Qualität bestimmt werden kann, insbesondere durch das implantierte Unterstützungssystem selbst.

Bei der hier vorgeschlagenen Lösung kann in besonders vorteilhafter Weise eine Korrelation zwischen gemessenem (Haupt-)Peak im Doppler-Frequenzspektrum und angelegter Pulswiederholrate (PRF) genutzt werden. Wird eine Messung mehrfach mit verschiedenen PRFs durchgeführt, liegen die bestimmbaren Maxima des jeweiligen Messzyklus mit veränderter PRF an einer anderen Position im Frequenzspektrum. Durch eine Variation der PRF-Werte kann so in vorteilhafter Weise ein Gleichungssystem aufgestellt werden, mithilfe dessen Doppeldeutigkeiten eliminiert und eineindeutige Lösungen ermittelt werden können. Dies ermöglicht in besonders vorteilhafter Weise die Berechnung der Hauptgeschwindigkeitskomponente einer Blutströmung trotz sog. "spectrum wrapping" bzw. Aliasing.

In Schritt a) erfolgt ein Durchführen einer ersten gepulsten Dopplermessung mit einer ersten Pulswiederholrate (erste PRF bzw. $PRF_1$) mittels eines Ultraschallsensors des Unterstützungssystems. Zur Durchführung der gepulsten Dopplermessung wird insbesondere das gepulste Doppler-(engl.: Pulsed Wave Doppler; kurz: PWD)-Verfahren eingesetzt. Insbesondere wird in Schritt a) ein erster PWD-Messzyklus durchlaufen.

In Schritt b) erfolgt ein Durchführen einer zweiten gepulsten Dopplermessung mit einer zweiten Pulswiederholrate (zweite PRF bzw. $PRF_2$) mittels des Ultraschallsensors des Unterstützungssystems. Hierbei unterscheidet sich die zweite Pulswiederholrate von der ersten Pulswiederholrate. Beispielsweise ist die zweite Pulswiederholrate größer oder kleiner als die erste Pulswiederholrate. Dies bedeutet mit anderen Worten insbesondere, dass es sich bei der ersten Pulswiederholrate und der zweiten Pulswiederholrate um voneinander verschiedene Pulswiederholraten handelt. Insbesondere wird in Schritt b) ein zweiter PWD-Messzyklus durchlaufen. Weiterhin bevorzugt erfolgt in den Schritten a) und b) eine Wiederholung eines PWD-Messzyklus mit verschiedenen, festgelegten PRF-Werten, z. B. 20 kHz und 25 kHz.

Es können auch weitere (gepulste) Dopplermessungen, beispielsweise eine dritte, vierte und/oder fünfte Dopplermessung durchgeführt werden. Für diese können die im Zusammenhang mit der ersten und der zweiten gepulsten Dopplermessung ausgeführten Sachverhalte entsprechend gelten. Insbesondere weisen alle diese gepulsten Dopplermessungen voneinander verschiedene Pulswiederholraten auf. Wenn weitere Dopplermessungen durchgeführt werden, können diese (ggf. nach Bedarf) in das Ermitteln in Schritt c) mit einfließen. Dies bedeutet mit anderen Worten insbesondere, dass das Ermitteln der Strömungsgeschwindigkeit auch unter Verwendung von Messergebnissen der ersten gepulsten Dopplermessung, der zweiten gepulsten Dopplermessung und weiteren gepulsten Dopplermessungen (soweit vorhanden) durchgeführt werden kann. Vorzugsweise ist die Anzahl der Dopplermessungen jedoch gerade so groß, dass mittels des Verfahrens eine eindeutige Strömungsgeschwindigkeit ermittelt werden kann, was hier in besonders vorteilhafter Weise bereits mit zwei Dopplermessungen erreicht werden kann.

Ein PWD-Messzyklus umfasst insbesondere eine Abfolge einer (definierten) Anzahl an nacheinander ausgesendeten Ultraschallimpulsen. Die mit unterschiedlichen PRFs durchgeführten Dopplermessungen der Schritte a) und b) werden grundsätzlich bezüglich derselben Fluidströmung, etwa in demselben Beobachtungsfenster bzw. in demselben Messbereich durchgeführt. Dies bedeutet mit anderen Worten insbesondere, dass die erste gepulste Dopplermessung und die zweite gepulste Dopplermessung innerhalb desselben Beobachtungsfensters bzw. Messbereichs erfolgen. Darüber hinaus ist es vorteilhaft, wenn die zwei gepulsten Dopplermessungen zeitlich unmittelbar aufeinander folgen bzw. kein signifikanter zeitlicher Abstand zwischen den beiden Messungen liegt.

In Schritt c) erfolgt ein Ermitteln der Strömungsgeschwindigkeit unter Verwendung von Messergebnissen der ersten gepulsten Dopplermessung und der zweiten gepulsten Dopplermessung. Die Messergebnisse sind in der Regel mehrdeutig. Diese Mehrdeutigkeit lässt sich insbesondere mit der hier in der Regel vorliegenden Verletzung des Nyquist-Abtasttheorems erklären. Diese Verletzung des Nyquist-Abtasttheorems wird insbesondere dadurch verursacht, dass in dem Unterstützungssystem zwischen Ultraschallsensor und Beobachtungsfenster bzw. Messbereich vergleichsweise lange Signallaufzeiten bestehen und bei den gepulsten Dopplermessungen in der Regel ein neuer Ultraschall-Impuls erst ausgesendet wird, wenn ein Echo eines unmittelbar zuvor ausgesendeten Ultraschall-Impulses empfangen wurde.

Das Ermitteln der Strömungsgeschwindigkeit kann beispielsweise derart erfolgen, dass zunächst ein erstes Doppler-Frequenzspektrum auf Basis der ersten gepulsten Dopplermessung und ein zweites Doppler-Frequenzspektrum auf Basis der zweiten gepulsten Dopplermessung erfasst wird. Dies bedeutet mit anderen Worten insbesondere, dass zunächst eine Berechnung des (jeweiligen) Doppler-Frequenzspektrums für jede PRF-Messung erfolgt. Weiterhin kann beispielsweise eine erste Hauptfrequenzkomponente des ersten Doppler-Frequenzspektrums und eine zweite Hauptfrequenzkomponente des zweiten Doppler-Frequenzspektrums ermittelt werden. Dies bedeutet mit anderen Worten insbesondere, dass eine Ermittlung der Hauptfrequenzkomponente des (jeweiligen) Doppler-Spektrums (z. B. einfacher Frequenzpeak oder ein sog. "template matching" der erwarteten Frequenzverteilung) erfolgt.

Auf Basis der ersten Hauptfrequenzkomponente und der zweiten Hauptfrequenzkomponente kann beispielhaft ein Gleichungssystem aufgestellt werden. Das Gleichungssystem kann beispielsweise durch Aufstellen und Lösen einer daraus resultierenden linearen Diophantischen Gleichung (z. B. lösbar durch Bezout Koeffizienten) gelöst werden. Aus dieser Lösung kann die tatsächliche bzw. eindeutige Doppler-Frequenz bestimmt werden. Mittels dieser (eindeutigen) Doppler-Frequenz kann in bekannter Art und Weise die Strömungsgeschwindigkeit berechnet werden.

Nach einer vorteilhaften Ausgestaltung wird vorgeschlagen, dass zumindest in einem der beiden Schritte a) und b) ein neuer Ultraschall-Impuls erst ausgesendet wird, wenn ein Echo eines unmittelbar zuvor ausgesendeten Ultraschall-Impulses empfangen wurde. Bevorzugt wird in beiden Schritten a) und b) jeweils ein neuer Ultraschall-Impuls erst ausgesendet, wenn ein Echo eines unmittelbar zuvor ausgesendeten Ultraschall-Impulses empfangen wurde. Besonders bevorzugt wird ein neuer Ultraschall-Impuls erst ausgesendet, wenn alle (signifikanten) Echos eines unmittelbar zuvor ausgesendeten Ultraschall-Impulses empfangen wurden.

Nach einer vorteilhaften Ausgestaltung wird vorgeschlagen, dass $PRF_1$ oder $PRF_2$ kleiner ist als das Zweifache einer maximal auftretenden Doppler-Verschiebung. Vorzugsweise ist die maximale Pulswiederholrate der gepulsten Dopplermessungen kleiner als die maximal auftretende bzw. zu erwartende Doppler-Verschiebung. Wenn die maximale Pulswiederholrate kleiner ist als das Zweifache der maximal auftretenden Doppler-Verschiebung, erfolgt grundsätzlich eine Verletzung des Nyquist-Abtasttheorems. Diese Verletzung kann jedoch erforderlich sein, um in einem vaskulären Unterstützungssystem ein PWD-Verfahren ausführen zu können.

Nach einer vorteilhaften Ausgestaltung wird vorgeschlagen, dass in Schritt c) eine Korrelation zwischen einer erfassten Hauptfrequenzkomponente des Doppler-Frequenzspektrums (insb. Peak im Doppler-Beitrag zum Frequenzspektrums) einer gepulsten Dopplermessung und der für diese Dopplermessung angelegten Pulswiederholrate verwendet wird. Die Hauptfrequenzkomponente des (jeweiligen) Doppler-Frequenzspektrums ist grundsätzlich die Frequenzkomponente, die charakteristisch für die gemessene Strömungsgeschwindigkeit ist. Die Hauptfrequenzkomponente ist insbesondere ein (lokales) Maximum, ein (lokaler) Ausschlag bzw. ein (Neben-)Peak im Doppler-Frequenzspektrum ("im Doppler-Frequenzspektrum" ist hier insbesondere eine Kurzform für "im Doppler-Beitrag zum Frequenzspektrum"; Dies soll verdeutlichen, dass mit dem Peak nicht der (immer größere) Trägerstrich im Spektrum gemeint ist.). Bevorzugt wird in Schritt c) eine Korrelation zwischen einem erfassten Peak im Doppler-Frequenzspektrum einer gepulsten Dopplermessung und der für diese Dopplermessung angelegten Pulswiederholrate verwendet. Weiterhin bevorzugt werden für diese Korrelation jeweils der erste und der zweite gepulste Dopplermesszyklus verwendet bzw. aufgestellt.

Wobei im Schritt c) ein lineares Gleichungssystem gelöst wird, in dem eine Dopplerverschiebung als Funktion von Hauptfrequenzkomponenten der ersten gepulsten Dopplermessung und der zweiten gepulsten Dopplermessung dargestellt wird. Die Dopplerverschiebung kann auch als Doppler-Frequenz (Formelzeichen df bzw. $\Delta$f) bezeichnet werden. Bevorzugt wird in dem linearen Gleichungssystem die Dopplerverschiebung als Funktion der Hauptfrequenzkomponenten und der (voneinander verschiedenen) Pulswiederholraten der ersten gepulsten Dopplermessung und der zweiten gepulsten Dopplermessung dargestellt. Die Anzahl der Gleichungen in dem Gleichungssystem entspricht in der Regel der Anzahl der durchgeführten Dopplermessungen. Besonders bevorzugt umfasst das lineare Gleichungssystem zwei lineare Gleichungen. Die erste lineare Gleichung gibt dabei die Dopplerverschiebung vorzugsweise als Funktion der Hauptfrequenzkomponente des ersten Doppler-Frequenzspektrums und/oder der ersten Pulswiederholrate an. Die zweite lineare Gleichung gibt dabei die Dopplerverschiebung vorzugsweise als Funktion der Hauptfrequenzkomponente des zweiten Doppler-Frequenzspektrums und/oder der zweiten Pulswiederholrate an.

Auf Basis des linearen Gleichungssystems wird eine lineare Diophantische Gleichung aufgestellt. Eine lineare diophantische Gleichung ist eine Gleichung der Form $a_1x_1 + a_2x_2 + a_3x_3 + \ldots + a_nx_n + c = 0$ mit ganzzahligen Koeffizienten ai, bei der man sich insbesondere nur für ganzzahlige Lösungen interessiert. Linear bedeutet, dass die Variablen xi nicht in Potenzen größer eins auftreten. Die lineare Diophantische Gleichung kann beispielsweise so aufgestellt werden, dass die beispielhaft zwei linearen Gleichungen nach der Doppler-Verschiebung aufgelöst und anschließend gleichgesetzt werden.

Nach einer weiteren vorteilhaften Ausgestaltung wird vorgeschlagen, dass die lineare Diophantische Gleichung unter Verwendung von Bezout Koeffizienten oder einer Exhaustionsmethode gelöst wird. Die sog. Bezout Koeffizienten können insbesondere dadurch ermittelt werden, dass die Gleichung $n_1 \cdot PRF_1 + n_2 \cdot PRF_2 = ggT(PRF_1, PRF_2)$ gelöst wird. Sie besagt insbesondere, dass sich der größte gemeinsame Teiler ggT zweier ganzzahliger Zahlen wie z. B. $PRF_1$ und $PRF_2$ als Linearkombination ganzzahliger Koeffizienten $n_1$ und $n_2$ darstellen lässt. Die so ermittelten Bezout Koeffizienten dienen insbesondere der Lösung der aufgestellten Diophantischen Gleichung. Die Exhaustionsmethode kann auch als sog. "Brute-Force"-Methode bezeichnet werden. Diese beschreibt eine Lösungsmethode, die auf dem Ausprobieren aller möglichen (oder zumindest vieler möglicher) Fälle beruht.

Nach einer vorteilhaften Ausgestaltung wird vorgeschlagen, dass unter Verwendung der Strömungsgeschwindigkeit ein Fluid-Volumenstrom durch das Unterstützungssystem ermittelt wird. Dies betrifft mit anderen Worten insbesondere einen Fluid-Volumenstrom der (nur) durch das Unterstützungssystems selbst, beispielsweise durch eine (Einlauf-)Kanüle des Unterstützungssystems hindurch fließt. Bei diesem Fluid-Volumenstrom handelt es sich üblicherweise um den sog. Pumpenvolumenstrom ($Q_p$), der nur den Fluss durch das Unterstützungssystem selbst quantifiziert. Ist dieser Wert zusätzlich zu dem Gesamtvolumenstrom bzw. Herz-Zeit-Volumen ($Q_{HZV}$) bekannt, so kann aus dem Verhältnis von $Q_p$ zu $Q_{HZV}$ (d. h. $Q_p/Q_{HZV}$) der sogenannte Unterstützungsgrad berechnet werden. Zur Bestimmung des Fluid-Volumenstroms kann die ermittelte Strömungsgeschwindigkeit beispielsweise mit einem durchströmbaren Querschnitt des Unterstützungssystems, insbesondere einem durchströmbaren Kanülen-Querschnitt, multipliziert werden.

Nach einem weiteren Aspekt wird ein implantierbares, vaskuläres Unterstützungssystem mit einem Ultraschallsensor und mit einer Verarbeitungseinheit vorgeschlagen, wobei

- der Ultraschallsensor zur Durchführung von gepulsten Dopplermessungen mit unterschiedlichen Pulswiederholraten $PRF_1 < PRF_2$ eingerichtet ist, und
- die Verarbeitungseinheit zum Ermitteln einer Strömungsgeschwindigkeit eines durch das Unterstützungssystem strömenden Fluids unter Verwendung von Messergebnissen der gepulsten Dopplermessungen mit unterschiedlichen Pulswiederholraten dient,

- das Ermitteln der Strömungsgeschwindigkeit unter Verwendung von Messergebnissen der ersten gepulsten Dopplermessung und der zweiten gepulsten Dopplermessung erfolgt, indem für ganzzahliges $n_1$, $n_2$ und einer Hauptkomponente $f_1$ der ersten gepulsten Dopplermessung und einer Hauptkomponente $f_2$ der zweiten gepulsten Dopplermessung folgende lineare Diophantische Gleichung

$$n_1 \cdot PRF_1 - n_2 \cdot PRF_2 = f_1 - f_2$$

für folgende Randbedingung

$$v < \frac{a \cdot PRF_1 \cdot c_0}{2 f_0}$$

unter der Annahme gelöst wird:

$$-|a| \leq n_1 \leq |a| \text{ und } -|b| \leq n_2 \leq |b|,$$

wobei

$$a := \frac{PRF_2}{2 \cdot ggT(PRF_1, PRF_2)} \text{ und } b := \frac{PRF_1}{2 \cdot ggT(PRF_1, PRF_2)},$$

und wobei $f_0$ die Ultraschall-Sendefrequenz des Ultraschallsensors und $c_0$ die Schallgeschwindigkeit in dem Fluid ist.

Bei dem Unterstützungssystem handelt es sich vorzugsweise um ein linksventrikuläres Herzunterstützungssystem (LVAD) bzw. ein perkutanes, minimalinvasives Linksherz-Unterstützungssystem. Weiterhin bevorzugt ist dieses voll-implantierbar. Das bedeutet mit anderen Worten insbesondere, dass die zur Erfassung erforderlichen Mittel, insbesondere der Ultraschallsensor, sich vollständig im Körper des Patienten befinden und dort verbleiben. Das Unterstützungssystem kann auch mehrteilig bzw. mit mehreren, beabstandet voneinander anordenbaren Komponenten ausgeführt sein, sodass beispielsweise der Ultraschallsensor und die Verarbeitungseinheit (Messeinheit) durch ein Kabel separiert voneinander angeordnet sein können. Bei der mehrteiligen Ausführung kann die separat von dem Ultraschallsensor angeordnete Verarbeitungseinheit ebenfalls implantiert oder aber außerhalb des Körpers des Patienten angeordnet werden. Es ist jedenfalls nicht zwingend erforderlich, dass auch die Verarbeitungseinheit im Körper des Patienten angeordnet wird. Beispielsweise kann das Unterstützungssystem so implantiert werden, dass die Verarbeitungseinheit auf der Haut des Patienten bzw. außerhalb des Körpers des Patienten angeordnet wird und eine Verbindung zu dem im Körper angeordneten Ultraschallsensor hergestellt wird. Besonders bevorzugt ist das Unterstützungssystem so eingerichtet bzw. dazu geeignet, dass es zumindest teilweise in einem Ventrikel, bevorzugt dem linken Ventrikel eines Herzens und/oder einer Aorta, insbesondere in Aortenklappenposition angeordnet werden kann.

Weiterhin bevorzugt umfasst das Unterstützungssystem eine Kanüle, insbesondere Einlaufkanüle, eine Strömungsmaschine, wie etwa eine Pumpe und/oder einen Elektromotor. Der Elektromotor ist dabei regelmäßig ein Bestandteil der Strömungsmaschine. Die (Einlauf-)Kanüle ist vorzugsweise so eingerichtet, dass sie im implantierten Zustand Fluid aus einem (linken) Ventrikel eines Herzens hin zu der Strömungsmaschine führen kann. Das Unterstützungssystem ist vorzugsweise länglich und/oder schlauchartig gebildet. Bevorzugt sind die Kanüle und die Strömungsmaschine im Bereich einander gegenüberliegender Enden des Unterstützungssystems angeordnet.

Es ist insbesondere genau bzw. nur ein Ultraschalsensor vorgesehen. Der Ultraschallsensor weist bevorzugt genau bzw. nur ein Ultraschall-Wandlerelement auf. Dies ist insbesondere dann ausreichend für eine Dopplermessung, wenn dabei das PWD-Verfahren zum Einsatz kommt.

Vorteilhafterweise ist das Unterstützungssystem zur Durchführung eines hier vorgeschlagenen Verfahrens eingerichtet.

Nach einem weiteren Aspekt wird eine Verwendung von gepulsten Dopplermessungen mit unterschiedlichen Pulswiederholraten zum Korrigieren eines mehrdeutigen Messergebnisses eines Ultraschallsensors eines implantierten, vaskulären Unterstützungssystems vorgeschlagen. Bevorzugt wird zumindest ein hier vorgeschlagenes Verfahren oder ein hier vorgeschlagenes Unterstützungssystem zum Korrigieren eines mehrdeutigen Messergebnisses des Ultraschallsensors verwendet.

Die im Zusammenhang mit dem Verfahren erörterten Details, Merkmale und vorteilhaften Ausgestaltungen können entsprechend auch bei dem hier vorgestellten Unterstützungssystem und/oder der Verwendung auftreten und umgekehrt. Insoweit wird auf die dortigen Ausführungen zur näheren Charakterisierung der Merkmale vollumfänglich Bezug genommen.

Die hier vorgestellte Lösung sowie deren technisches Umfeld werden nachfolgend anhand der Figuren näher erläutert. Es ist darauf hinzuweisen, dass die Erfindung durch die gezeigten Ausführungsbeispiele nicht beschränkt werden soll. Insbesondere ist es, soweit nicht explizit anders dargestellt, auch möglich, Teilaspekte der in den Figuren erläuterten Sachverhalte zu extrahieren und mit anderen Bestandteilen und/oder Erkenntnissen aus anderen Figuren und/oder der vorliegenden Beschreibung zu kombinieren. Es zeigen schematisch:

Fig. 1    ein implantiertes, vaskuläres Unterstützungssystem in einem Herz,

Fig. 2    das Unterstützungssystem aus Fig. 1,

Fig. 3    einen Ablauf eines hier vorgestellten Verfahrens bei einem regulären Betriebsablauf,

Fig. 4    ein beispielhaftes Doppler-Frequenzspektrum, und

Fig. 5    ein weiteres beispielhaftes Doppler-Frequenzspektrum.

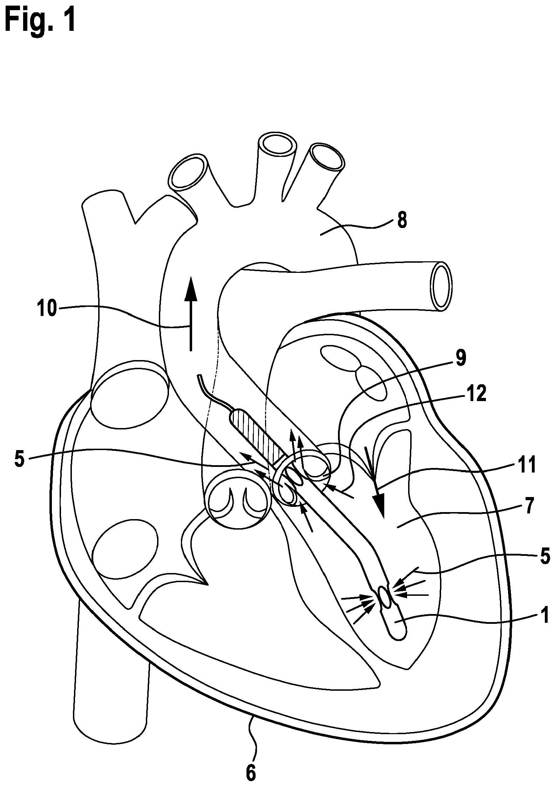

Fig. 1 zeigt schematisch ein implantiertes, vaskuläres (hier: ventrikuläres) Unterstützungssystem 1 in einem Herz 6. Das Unterstützungssystem 1 unterstützt das Herz 6, indem es dazu beiträgt Blut aus dem (linken) Ventrikel 7 in die Aorta 8 zu fördern. Das Unterstützungssystem 1 wird hierzu in der Aortenklappe 9 verankert, wie Fig. 1 beispielhaft verdeutlicht. Bei einem Unterstützungsgrad von 100% fördert das Unterstützungssystem 1 (LVAD) den kompletten Blutvolumenstrom. Der Unterstützungsgrad beschreibt den Anteil des durch ein Fördermittel wie etwa eine Pumpe des Unterstützungssystems 1 bzw. durch das Unterstützungssystem 1 hindurch geförderten Volumenstrom zum Gesamtvolumenstrom an Blut vom Ventrikel 7 hin zur Aorta 8.

Bei einem Unterstützungsgrad von 100% sind demnach der Fluid-Gesamtvolumenstrom 10 aus dem Ventrikel 7, der Herzklappenvolumenstrom 11 in den Ventrikel 7 sowie der Fluid-Volumenstrom 5 durch das Unterstützungssystem 1 identisch. Der Aortenklappen- bzw. Bypass-Volumenstrom 12 (Formelzeichen: $Q_a$) ist folglich dabei Null. Der Fluid-Gesamtvolumenstrom 10 kann auch als (gesamtes) Herz-Zeit-Volumen (HZV, Formelzeichen: $Q_{HZV}$) beschrieben werden. Der Fluid-Volumenstrom 5 kann auch als sog. Pumpenvolumenstrom (Formelzeichen: $Q_p$) bezeichnet werden der nur den Fluss durch das Unterstützungssystem 1 selbst quantifiziert. Der Unterstützungsgrad kann somit aus dem Verhältnis $Q_p/Q_{HZV}$ berechnet werden.

Bei geringeren Unterstützungsgraden und gesünderen Herzen mit starker Ventrikelkontraktion erfüllt das Herz 6 seine Funktion weiterhin zu einem gewissen Anteil, sodass während der Systole (Herzmuskel zieht sich zusammen und verdrängt durch die Volumenabnahme des Ventrikels 7 Blut in die Aorta 8) ein pulsatiler Volumenstromanteil 12 (Bypass) durch die Herz- bzw. Aortenklappe 9 entsteht. Gleichzeitig sinkt die Druckdifferenz über das Unterstützungssystem 1, insbesondere über die üblicherweise vorgesehene Pumpe (hier nicht dargestellt) des Unterstützungssystems 1, sodass entsprechend das Unterstützungssystem 1 während der Systole ebenfalls einen gestiegenen Fluid-Volumenstrom 5 fördert.

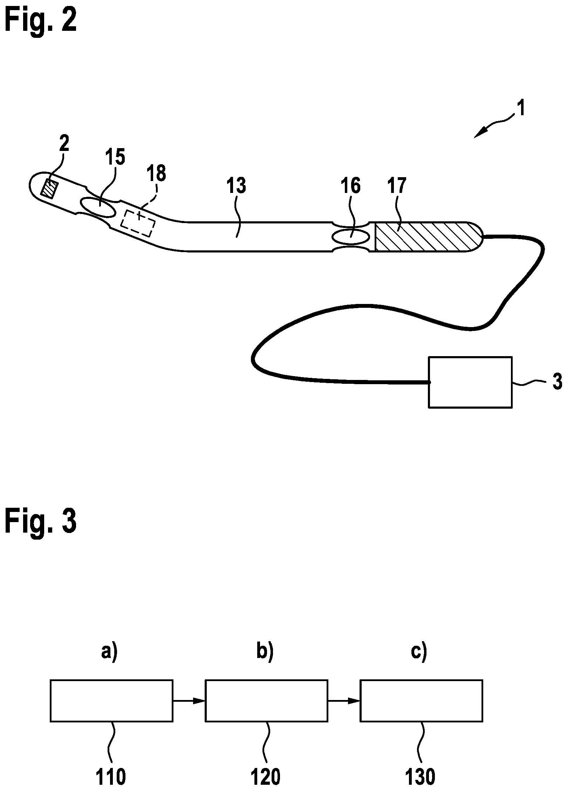

Fig. 2 zeigt schematisch das Unterstützungssystem 1 aus Fig. 1. Das Unterstützungssystem 1 umfasst einen Ultraschallsensor 2, eingerichtet zur Durchführung von gepulsten Dopplermessungen mit unterschiedlichen Pulswiederholraten und eine Verarbeitungseinheit 3, eingerichtet zum Ermitteln einer Strömungsgeschwindigkeit eines durch das Unterstützungssystem 1 strömenden Fluids (hier: Blut) unter Verwendung von Messergebnissen der gepulsten Dopplermessungen mit unterschiedlichen Pulswiederholraten.

In Fig. 2 ist zudem beispielhaft veranschaulicht, dass der Ultraschallsensor 2 in der Spitze einer Kanüle 13 des Unterstützungssystems 1 integriert sein kann. Der Ultraschallsensor 2 trägt zur Bestimmung der Strömungsgeschwindigkeit (Betrag und zumindest eine Richtung) eines durch das Unterstützungssystem 1 strömenden Fluids bzw. des Fluid-Volumenstroms 5, der auch als Pumpenvolumenstrom ($Q_p$) bezeichnet wird, bei. Hierzu ist der Ultraschallsensor 2 zur Durchführung von gepulsten Dopplermessungen in dem Fluid innerhalb der Kanüle 13 eingerichtet. Das Fluid kann durch eine oder mehrere Eintrittsöffnungen 15 (aus dem Ventrikel 7) in das Innere der Kanüle 13 eintreten und durch eine oder mehrere Austrittsöffnungen 16 (in die Aorta 8) austreten. Zur Unterstützung der Fluidströmung durch das Unterstützungssystem 1, insbesondere durch die Kanüle 13 weist das Unterstützungssystem 1 hier eine Strömungsmaschine 17 auf. Die Strömungsmaschine 17 ist in der Regel in der Art einer Pumpe gebildet. Ferner ist in Fig. 2 auch ein Beobachtungsfenster bzw. ein Messbereich 18 des Ultraschallsensors 2 beispielhaft eingetragen.

Fig. 3 zeigt schematisch einen Ablauf eines hier vorgestellten Verfahrens bei einem regulären Betriebsablauf. Das Verfahren dient zur Bestimmung einer Strömungsgeschwindigkeit eines durch ein implantiertes, vaskuläres Unterstützungssystem 1 (vgl. Fig. 1, 2) strömenden Fluids. Die dargestellte Reihenfolge der Verfahrensschritte a), b) und c) mit den Blöcken 110, 120 und 130 ist lediglich beispielhaft. In Block 110 erfolgt ein Durchführen einer ersten gepulsten Dopplermessung mit einer ersten Pulswiederholrate mittels eines Ultraschallsensors 2 des Unterstützungssystems 1. In Block 120 erfolgt ein Durchführen einer zweiten gepulsten Dopplermessung mit einer zweiten Pulswiederholrate mittels des Ultraschallsensors 2 des Unterstützungssystems 1, wobei sich die zweite Pulswiederholrate von der ersten Pulswiederholrate unterscheidet. In Block 130 erfolgt ein Ermitteln der Strömungsgeschwindigkeit unter Verwendung von Messergebnissen der ersten gepulsten Dopplermessung und der zweiten gepulsten Dopplermessung.

Für eine exemplarische Darstellung des Verfahrens werden folgende Parameter angenommen:

- Durchmesser Einlauf- bzw. Messbereich z. B. 5 mm,
- Maximal zu messender Blutfluss z. B. Q = 9 l/min,
- Resultierende max. Blutflussgeschwindigkeit: $v_{Blut,max}$ = 7,64 m/s,
- Schallgeschwindigkeit im Blut z. B. $c_{Blut}$ = 1540 m/s,
- Ultraschallfrequenz z. B. fo = 6 MHz,
- Abstand Ultraschallelement zu Beginn Betrachtungsfenster z. B. 25 mm,
- Anzahl Ultraschall-Schwindungszyklen pro ausgesendetem Ultraschall-PWD-Impuls z. B. 10,
- Resultierende Burstlänge (in Strecke): $l_{Burst} = c_0 \times 10/f_0 = 2{,}57$ mm,
- Resultierende maximale Ausbreitungsstrecke Ultraschall-Burst: d = 55,13 mm.

Aus diesen Angaben ergibt sich für eine Messung direkt in Abstrahlrichtung (Flussrichtung entspricht Hauptabstrahlrichtung; $\alpha = 0$) folgende (zu erwartende) maximale Doppler-Verschiebung:

$$df = \frac{2 \cdot v_{Blut,max} \cdot f_0}{c_0} = \frac{2 \cdot 7,64\frac{m}{s} \cdot 6MHz}{1540\frac{m}{s}} = 59,53kHz$$

$$(1)$$

Die Messung soll als gepulste Dopplermessung ausgeführt werden, bei welcher ein neuer Ultraschall-Impuls erst ausgesendet wird, wenn ein Echo eines unmittelbar zuvor ausgesendeten Ultraschall-Impulses abgeklungen ist. Die Wahl der hierfür zu verwendenden Pulswiederholrate (PRF) wird nachstehend erläutert.

Unter Beachtung des (Nyquist-)Abtasttheorems (welches bei der hier vorgestellten Lösung jedoch gerade nicht beachtet werden muss bzw. wird) würde eine maximale Dopplerfrequenz von 59,53 kHz bedeuten, dass eine minimale Pulswiederholrate bzw. minimale Pulswiederholfrequenz von

$$PRF_{min} = 2 \cdot df = 119,06kHz\,. \qquad (2)$$

eingehalten werden müsste.

Bei den hier im Fokus stehenden implantierten, vaskulären Unterstützungssystemen ergibt sich jedoch aus der geometrischen Betrachtung (maximale Ausbreitungsstrecke des Ultraschallimpulses) bzw. den geometrischen Randbedingungen im Unterstützungssystem und der daraus resultierenden Laufzeit aller relevanten Signalanteile die folgende maximale Pulswiederholrate PRF-$_{max}$:

$$PRF_{max} = \frac{c_{Blut}}{d} = 27,93kHz \qquad (3)$$

Somit ist die maximale Pulswiederholrate der gepulsten Dopplermessungen hier (bzw. für die im Fokus stehenden Unterstützungssysteme) kleiner als das Zweifache der maximal auftretenden Doppler-Verschiebung.

Diese Randbedingungen führen zu einer Verletzung des Abtasttheorems und folglich zu einer Mehrdeutigkeit der Messergebnisse, die durch eine Auswertung wie in den folgenden Abschnitten beschrieben behoben werden kann.

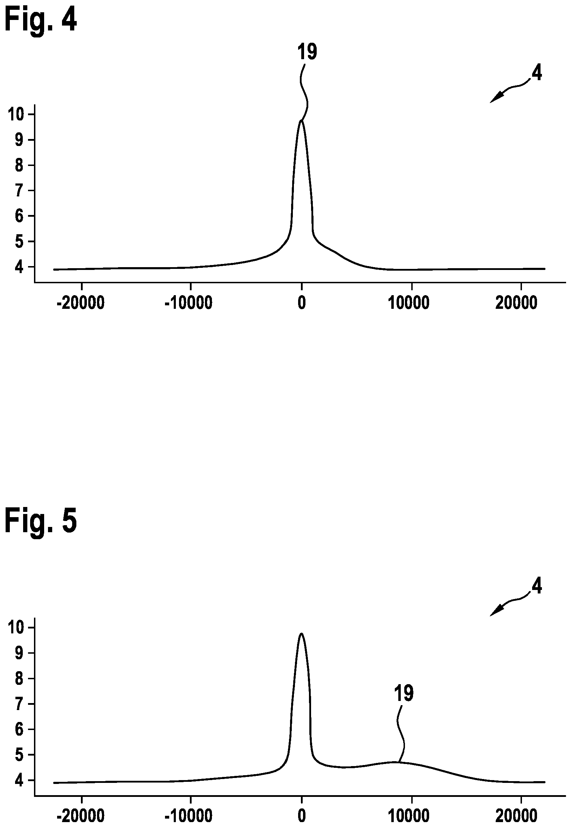

Zuvor wird jedoch zur Veranschaulichung der bei diesen Randbedingungen entstehenden Problematik die dabei auftretende Mehrdeutigkeit in den Fig. 4 und 5 veranschaulicht (die mit der hier vorgestellten Lösung aufgelöst werden kann). Fig. 4 zeigt schematisch ein beispielhaftes Doppler-Frequenzspektrum 4. Fig. 4 zeigt eine Doppler-Verschiebung bei einer Pulswiederholrate von ca. 25 kHz. Die Hauptfrequenzkomponente 19 (Peak) liegt unter der Trägerfrequenz bei ca. 0 Hz. Fig. 5 zeigt schematisch ein weiteres beispielhaftes Doppler-Frequenzspektrum 4. Fig. 5 zeigt eine Doppler-Verschiebung bei einer Pulswiederholrate von ca. 20 kHz. Die Hauptfrequenzkomponente 19 (Peak) liegt bei ca. +8 kHz.

In den nachfolgenden Abschnitten wird eine beispielhafte Auswertung der mehrdeutigen Messergebnisse im Sinne der hier vorgeschlagenen Lösung beschrieben.

Zwei Messzyklen (Abfolge einer definierten Anzahl an nacheinander ausgesendeten Ultraschallimpulsen) bei unterschiedlichen PRFs werden (bezüglich derselben Fluidströmung, etwa in demselben Beobachtungsfenster) aufgenommen. Die tatsächliche Doppler-Verschiebung kann als Funktion der resultierenden Hauptfrequenzkomponenten 19, hier Peaks $f_1$ und $f_2$, dargestellt werden:

$$df = f_1 + n_1 \cdot PRF_1 \qquad (4)$$

$$df = f_2 + n_2 \cdot PRF_2 \qquad (5)$$

Dies veranschaulicht beispielhaft, wie ein lineares Gleichungssystem aufgestellt werden kann, in dem die Dopplerverschiebung df als Funktion von Hauptfrequenzkomponenten 19, hier Peaks $f_1$ und $f_2$, der ersten gepulsten Dopplermessung und der zweiten gepulsten Dopplermessung dargestellt wird. Zudem veranschaulicht dies beispielhaft eine Korrelation zwischen einer erfassten Hauptfrequenzkomponente 19 des Doppler-Frequenzspektrums, hier eines Peaks im Doppler-Frequenzspektrum, einer gepulsten Dopplermessung und der für diese Dopplermessung angelegten Pulswiederholrate.

Durch das Auflösen beider Gleichungen nach der Doppler-Verschiebung df und anschließendem Gleichsetzen entsteht die nachfolgende Diophantische Gleichung:

$$n_1 \cdot PRF_1 - n_2 \cdot PRF_2 = f_2 - f_1 \qquad (6)$$

Dies veranschaulicht beispielhaft, wie auf Basis des linearen Gleichungssystems eine lineare Diophantische Gleichung aufgestellt werden kann.

Mit der Schallgeschwindigkeit in Blut $c_{Blut}$, der Ultraschall-Sendefrequenz $f_0$, der Flussgeschwindigkeit des Blutes $v_{Blut}$ sowie einer ganzzahligen Pulswiederholrate, lässt sich für diese Gleichung eine eineindeutige Lösung finden.

$$-|a| \leq n_1 \leq |a| \quad \text{mit} \quad a = \frac{PRF_2}{2 \cdot ggT(PRF_1, PRF_2)} \qquad (7)$$

$$-|b| \leq n_2 \leq |b| \quad \text{mit} \quad b = \frac{PRF_1}{2 \cdot ggT(PRF_1, PRF_2)} \qquad (8)$$

$$v < \frac{a \cdot PRF_1 \cdot c_0}{2 \cdot f_0} \quad \text{mit} \quad PRF_1 < PRF_2 \qquad (9)$$

Der Operator ggT stellt dabei den größten gemeinsamen Teiler dar. Innerhalb dieser Bereiche kann diese Gleichung z. B. mit Hilfe von Bezout Koeffizienten oder mit einem Exhaustions-(engl.: brute force)-Ansatz gelöst werden.

Beispielhaft wird dieses Vorgehen mithilfe der Werte v = 8 m/s, $f_0$ = 4 MHz, $PRF_1$ = 11 kHz und $PRF_2$ = 19 kHz veranschaulicht. Bei diesen Werten werden die resultierenden Peaks bei $f_1$ = -2442 Hz und $f_2$ = 3558Hz detektiert. Die resultierende Diophantische Gleichung lautet:

$$n_1 \cdot 11 - n_2 \cdot 19 = 6 \qquad (10)$$

Der größte gemeinsame Teiler für diesen Fall ist 1 und die Bezout Koeffizienten lauten 7 und 4. Daraus ergeben sich folgende mögliche Lösungen:

$$n_1 = 6 \cdot 7 + m \cdot 19 \qquad = 4 + m \cdot 19 \qquad (11)$$

$$n_2 = 6 \cdot 4 + m \cdot 11 \qquad = 2 + m \cdot 11 \qquad (12)$$

Dies veranschaulicht beispielhaft, wie die lineare Diophantische Gleichung unter Verwendung von Bezout Koeffizienten gelöst werden kann.

Da nur für m = 0 eine eindeutige Lösung bestimmt werden kann, wird nur diese betrachtet. Daraus kann die nun nicht mehr mehrdeutige Frequenz der

Doppler-Verschiebung ermittelt werden. Beide Gleichungen liefern dasselbe Ergebnis.

$$df = f_1 + n_1 \cdot PRF_1 \qquad\qquad (13)$$
$$= -2442 Hz + 4 \cdot 11000 Hz \qquad = 41558 Hz \qquad (14)$$

$$df = f_2 + n_2 \cdot PRF_2 \qquad\qquad (15)$$
$$= 3558 Hz + 2 \cdot 19000 Hz \qquad = 41558 Hz \qquad (16)$$

Die Strömungsgeschwindigkeit des durch das Unterstützungssystem 1 strömenden Fluids (hier: Blut) kann auf dieser Basis über die Frequenzverschiebung durch den Doppler-Effekt berechnet werden:

$$df = f_0 \cdot \frac{2v}{c} \cdot \cos(\alpha)$$

Mit df der resultierenden (eindeutigen) Doppler-Frequenzverschiebung, $f_0$ der Frequenz des ausgesendeten Ultraschall-Impulses, v der (hier gesuchten) Flussgeschwindigkeit des Mediums, c der Schallgeschwindigkeit im Medium und $\alpha$ dem Winkel zwischen Ultraschall-Schallpfad und Hauptströmungsrichtung.

Bei einem (Herz-)Unterstützungssystem ist v gesucht, a, $f_0$ und c sind in der Regel (zumindest näherungsweise) bekannt. Die bei solchen (Herz-)Unterstützungssystemen wie vorstehend erörtert in der Regel auftretende Mehrdeutigkeit kann mittels der hier vorgeschlagenen Lösung besonders vorteilhaft kompensiert werden. Auf Basis der ermittelten Strömungsgeschwindigkeit kann unter Verwendung der (bekannten) geometrischen Randbedingungen im Unterstützungssystem (bekannter durchströmbarer Querschnitt des Mess- bzw. Beobachtungsbereichs) der Fluid-Volumenstrom durch das Unterstützungssystem ermittelt werden. Dieser Fluid-Volumenstrom kann zumindest näherungsweise zur Erfassung des tatsächlich durch ein (Herz-)Unterstützungssystem geförderten Blutvolumens beitragen. Die Kenntnis über das tatsächlich geförderte Blutvolumen eines Herzunterstützungssystems bzw. kardialen Unterstützungssystems ist medizinisch von großer Bedeutung, insbesondere zur Regelung des (implantierten) Unterstützungssystems.

Die hier vorgestellte Lösung ermöglich insbesondere einen oder mehrere der nachfolgenden Vorteile:

- Gepulste Doppler- bzw. PWD-basierte Flussgeschwindigkeits- bzw. Volumenflussmessung wird auch bei großem Abstand zwischen Messfenster und Ultraschallwandler ermöglicht.
- Auflösung der geometrisch bedingten Mehrdeutigkeit der Doppler-Verschiebung aufgrund von geometrischen Randbedingungen im Unterstützungssystem

**Patentansprüche**

1. Verfahren zur Bestimmung einer Strömungsgeschwindigkeit *v* eines durch ein implantiertes, vaskuläres Unterstützungssystem (1) strömenden Fluids, umfassend:

a) Das Durchführen einer ersten gepulsten Dopplermessung mit einer ersten Pulswiederholrate $PRF_1$ mittels eines Ultraschallsensors (2) des Unterstützungssystems (1),
**gekennzeichnet durch**
b) das Durchführen einer zweiten gepulsten Dopplermessung mit einer zweiten Pulswiederholrate $PRF_2 > PRF_1$ mittels des Ultraschallsensors (2) des Unterstützungssystems (1), wobei sich die zweite Pulswiederholrate von der ersten Pulswiederholrate unterscheidet, und

das Ermitteln der Strömungsgeschwindigkeit unter Verwendung von Messergebnissen der ersten gepulsten Dopplermessung und der zweiten gepulsten Dopplermessung, indem für ganzzahliges $n_1$, $n_2$ und einer Hauptkomponente $f_1$ der ersten gepulsten Dopplermessung und einer Hauptkomponente $f_2$ der zweiten gepulsten Dopplermessung folgende lineare Diophantische Gleichung

$$n_1 \cdot PRF_1 - n_2 \cdot PRF_2 = f_1 - f_2$$

für folgende Randbedingung

$$v < \frac{a \cdot PRF_1 \cdot c_0}{2f_0}$$

unter der Annahme gelöst wird:

$$-|a| \leq n_1 \leq |a|$$

und

$$-|b| \leq n_2 \leq |b|,$$

wobei

$$a := \frac{PRF_2}{2 \cdot ggT(PRF_1, PRF_2)}$$

und

$$b := \frac{PRF_1}{2 \cdot ggT(PRF_1, PRF_2)},$$

und wobei $f_0$ die Ultraschall-Sendefrequenz des Ultraschallsensors (2) und $c_0$ die Schallgeschwindigkeit in dem Fluid ist.

**2.** Verfahren nach Anspruch 1, **dadurch gekennzeichnet, dass** in einem der beiden Schritte a) und b) ein neuer Ultraschall-Impuls erst ausgesendet wird, wenn ein Echo eines unmittelbar zuvor ausgesendeten Ultraschall-Impulses empfangen wurde.

**3.** Verfahren nach Anspruch 1 oder 2, **dadurch gekennzeichnet, dass** $PRF_1$ oder $PRF_2$ kleiner ist als das Zweifache einer maximal auftretenden Doppler-Verschiebung.

**4.** Verfahren nach einem der vorhergehenden Ansprüche, **dadurch gekennzeichnet, dass** in Schritt c) eine Korrelation zwischen einer erfassten Hauptfrequenzkomponente (14) des Doppler-Frequenzspektrums (4) einer gepulsten Dopplermessung und der für diese Dopplermessung angelegten Pulswiederholrate verwendet wird.

**5.** Verfahren nach einem der Ansprüche 1 bis 4, **dadurch gekennzeichnet, dass** die lineare Diophantische Gleichung unter Verwendung von Bezout Koeffizienten oder einer Exhaustionsmethode gelöst wird.

6. Verfahren zur Bestimmung eines Fluidstroms durch ein implantiertes vaskuläres Unterstützungssystem (1), bei dem in einem Verfahren nach einem der Ansprüche 1 bis 5 die Strömungsgeschwindigkeit $v$ des Fluids in dem Unterstützungssystem bestimmt und daraus ein Fluid-Volumenstrom durch das Unterstützungssystem (1) ermittelt wird.

7. Implantierbares, vaskuläres Unterstützungssystem (1) mit einem Ultraschallsensor (2) und mit einer Verarbeitungseinheit (3),
   **dadurch gekennzeichnet, dass**

   der Ultraschallsensor (2) zur Durchführung von gepulsten Dopplermessungen mit unterschiedlichen Pulswiederholraten $PRF_1 < PRF_2$ eingerichtet ist, und
   die Verarbeitungseinheit (3) zum Ermitteln einer Strömungsgeschwindigkeit eines durch das Unterstützungssystem (1) strömenden Fluids unter Verwendung von Messergebnissen der gepulsten Dopplermessungen mit unterschiedlichen Pulswiederholraten dient, wobei
   das Ermitteln der Strömungsgeschwindigkeit unter Verwendung von Messergebnissen der ersten gepulsten Dopplermessung und der zweiten gepulsten Dopplermessung erfolgt, indem für ganzzahliges $n_1$, $n_2$ und einer Hauptkomponente $f_1$ der ersten gepulsten Dopplermessung und einer Hauptkomponente $f_2$ der zweiten gepulsten Dopplermessung folgende lineare Diophantische Gleichung

$$n_1 \cdot PRF_1 - n_2 \cdot PRF_2 = f_1 - f_2$$

für folgende Randbedingung

$$v < \frac{a \cdot PRF_1 \cdot c_0}{2f_0}$$

unter der Annahme gelöst wird:

$$-|a| \leq n_1 \leq |a|$$

und

$$-|b| \leq n_2 \leq |b|,$$

wobei

$$a := \frac{PRF_2}{2 \cdot ggT(PRF_1, PRF_2)}$$

und

$$b := \frac{PRF_1}{2 \cdot ggT(PRF_1, PRF_2)},$$

und wobei $f_0$ die Ultraschall-Sendefrequenz des Ultraschallsensors (2) und $c_0$ die Schallgeschwindigkeit in dem Fluid ist.

8. Unterstützungssystem nach Anspruch 7, **dadurch gekennzeichnet, dass** die lineare Diophantische Gleichung unter Verwendung von Bezout Koeffizienten oder einer Exhaustionsmethode gelöst wird.

9. Unterstützungssystem nach Anspruch 7 oder Anspruch 8, **dadurch gekennzeichnet, dass** die Verarbeitungseinheit (3) für das Bereitstellen eines aus der Strömungsgeschwindigkeit berechneten Fluidstroms ausgelegt ist.

**Claims**

1. Method for determining a flow rate *v* of a fluid flowing through an implanted vascular support system (1), comprising:

   (a) carrying out a first pulsed Doppler measurement at a first pulse repetition rate $PRF_1$ using an ultrasonic sensor (2) of the support system (1), **characterized by**
   (b) carrying out a second pulsed Doppler measurement at a second pulse repetition rate $PRF_2 > PRF_1$ using the ultrasonic sensor (2) of the support system (1), the second pulse repetition rate differing from the first pulse repetition rate, and

   ascertaining the flow rate using measurement results of the first pulsed Doppler measurement and the second pulsed Doppler measurement, by solving for integer $n_1$, $n_2$ and a main component $f_1$ of the first pulsed Doppler measurement and a main component $f_2$ of the second pulsed Doppler measurement the following linear Diophantine equation

   $$n_1 \cdot PRF_1 - n_2 \cdot PRF_2 = f_1 - f_2$$

   for the following boundary condition

   $$v < \frac{a \cdot PRF_1 \cdot c_0}{2f_0}$$

   assuming

   $$-|a| \leq n_1 \leq |a|$$

   and

   $$-|b| \leq n_2 \leq |b|,$$

   where

   $$a := \frac{PRF_2}{2 \cdot ggT(PRF_1, PRF_2)}$$

   and

   $$b := \frac{PRF_1}{2 \cdot ggT(PRF_1, PRF_2)},$$

   and where $f_0$ is the ultrasonic transmission frequency of the ultrasonic sensor (2) and $c_0$ is the speed of sound in the fluid.

2. Method according to claim 1, **characterized in that** in one of the two steps a) and b) a new ultrasonic pulse is transmitted only when an echo of an ultrasonic pulse transmitted immediately beforehand has been received.

3. Method according to either claim 1 or claim 2, **characterized in that** $PRF_1$ or $PRF_2$ is less than twice a maximum occurring Doppler shift.

4. Method according to any of the preceding claims, **characterized in that** in step c) a correlation between a detected main frequency component (14) of the Doppler frequency spectrum (4) of a pulsed Doppler measurement and the pulse repetition rate applied for this Doppler measurement is used.

5. Method according to any of claims 1 to 4, **characterized in that** the linear Diophantine equation is solved using Bezout coefficients or an exhaustion method.

6. Method for determining a fluid flow through an implanted vascular support system (1), in which the flow rate $v$ of the fluid in the support system is determined in a method according to any of claims 1 to 5 and a fluid volume flow through the support system (1) is ascertained therefrom.

7. Implantable, vascular support system (1) comprising an ultrasonic sensor (2) and comprising a processing unit (3), **characterized in that**

the ultrasonic sensor (2) is set up to carry out pulsed Doppler measurements at different pulse repetition rates $PRF_1 < PRF_2$ , and
the processing unit (3) serves to ascertain a flow rate of a fluid flowing through the support system (1) using measurement results of the pulsed Doppler measurements at different pulse repetition rates,
the flow rate being ascertained using measurement results of the first pulsed Doppler measurement and the second pulsed Doppler measurement, by solving for integer $n_1$, $n_2$ and a main component $f_1$ of the first pulsed Doppler measurement and a main component $f_2$ of the second pulsed Doppler measurement the following linear Diophantine equation

$$n_1 \cdot PRF_1 - n_2 \cdot PRF_2 = f_1 - f_2$$

for the following boundary condition

$$v < \frac{a \cdot PRF_1 \cdot c_0}{2 f_0}$$

assuming:

$$-|a| \leq n_1 \leq |a|$$

and

$$-|b| \leq n_2 \leq |b|,$$

where

$$a := \frac{PRF_2}{2 \cdot ggT(PRF_1, PRF_2)}$$

and

$$b := \frac{PRF_1}{2 \cdot ggT(PRF_1, PRF_2)},$$

and where $f_0$ is the ultrasonic transmission frequency of the ultrasonic sensor (2) and $c_0$ is the speed of sound in the fluid.

8. Support system according to claim 7, **characterized in that** the linear Diophantine equation is solved using Bezout coefficients or an exhaustion method.

9. Support system according to either claim 7 or claim 8, **characterized in that** the processing unit (3) is designed to provide a fluid flow calculated from the flow rate.

**Revendications**

1. Procédé permettant de déterminer une vitesse d'écoulement v d'un fluide s'écoulant à travers un système d'assistance (1) vasculaire implanté, comprenant :

(a) la réalisation d'une première mesure Doppler pulsée avec un premier taux de répétition des impulsions $PRF_1$ au moyen d'un capteur à ultrasons (2) du système d'assistance (1),
**caractérisé par**
(b) la réalisation d'une seconde mesure Doppler pulsée avec un second taux de répétition des impulsions $PRF_2$ > $PRF_1$ au moyen du capteur à ultrasons (2) du système d'assistance (1), le second taux de répétition des impulsions étant différent du premier taux de répétition des impulsions, et

la détermination de la vitesse d'écoulement à l'aide de résultats de mesure de la première mesure Doppler pulsée et de la seconde mesure Doppler pulsée en résolvant l'équation diophantienne linéaire suivante pour des nombres entiers $n_1$, $n_2$ et une composante principale $f_1$ de la première mesure Doppler pulsée et une composante principale $f_2$ de la seconde mesure Doppler pulsée

$$n_1 \cdot PRF_1 - n_2 \cdot PRF_2 = f_1 - f_2$$

pour les contraintes suivantes

$$v < \frac{a \cdot PRF_1 \cdot c_0}{2f_0}$$

en supposant que :

$$-|a| \leq n_1 \leq |a|$$

et

$$-|b| \leq n_2 \leq |b|,$$

où

$$a := \frac{PRF_2}{2 \cdot ggT(PRF_1, PRF_2)}$$

et

$$b := \frac{PRF_1}{2 \cdot ggT(PRF_1, PRF_2)},$$

et où $f_0$ représente la fréquence de transmission d'ultrasons du capteur à ultrasons (2) et $c_0$ représente la

vitesse du son dans le fluide.

2. Procédé selon la revendication 1, **caractérisé en ce que**, dans l'une des deux étapes a) et b), une nouvelle impulsion ultrasonore n'est émise que lorsqu'un écho d'une impulsion ultrasonore émise immédiatement auparavant a été reçu.

3. Procédé selon la revendication 1 ou 2, **caractérisé en ce que** $PRF_1$ ou $PRF_2$ est inférieur à deux fois un décalage Doppler maximal rencontré.

4. Procédé selon l'une des revendications précédentes, **caractérisé en ce que**, à l'étape c), une corrélation entre une composante de fréquence principale détectée (14) du spectre de fréquences Doppler (4) d'une mesure Doppler pulsée et le taux de répétition des impulsions appliqué pour ladite mesure Doppler est utilisée.

5. Procédé selon l'une des revendications 1 à 4, **caractérisé en ce que** l'équation diophantienne linéaire est résolue à l'aide de coefficients de Bézout ou d'une méthode d'exhaustion.

6. Procédé permettant de déterminer un écoulement de fluide à travers un système d'assistance (1) vasculaire implanté, dans lequel la vitesse d'écoulement v du fluide dans le système d'assistance est déterminée dans un procédé selon l'une des revendications 1 à 5 et un débit volumique de fluide à travers le système d'assistance (1) est ainsi déterminé.

7. Système d'assistance (1) vasculaire implantable comportant un capteur à ultrasons (2) et comportant une unité de traitement (3),
**caractérisé en ce que**

le capteur à ultrasons (2) est configuré pour réaliser des mesures Doppler pulsées avec différents taux de répétition des impulsions $PRF_1 < PRF_2$, et
l'unité de traitement (3) sert à déterminer une vitesse d'écoulement d'un fluide s'écoulant à travers le système d'assistance (1) à l'aide de résultats de mesure des mesures Doppler pulsées avec différents taux de répétition des impulsions,
la vitesse d'écoulement étant déterminée à l'aide de résultats de mesure de la première mesure Doppler pulsée et de la seconde mesure Doppler pulsée par la résolution de l'équation diophantienne linéaire suivante pour des nombres entiers $n_1$, $n_2$ et une composante principale $f_1$ de la première mesure Doppler pulsée et une composante principale $f_2$ de la seconde mesure Doppler pulsée

$$n_1 \cdot PRF_1 - n_2 \cdot PRF_2 = f_1 - f_2$$

pour les contraintes suivantes

$$v < \frac{a \cdot PRF_1 \cdot c_0}{2f_0}$$

en supposant que :

$$-|a| \leq n_1 \leq |a|$$

et

$$-|b| \leq n_2 \leq |b|,$$

où

$$a := \frac{PRF_2}{2 \cdot ggT(PRF_1, PRF_2)} \,.$$

et

$$b := \frac{PRF_1}{2 \cdot ggT(PRF_1, PRF_2)},$$

et où $f_0$ représente la fréquence de transmission d'ultrasons du capteur à ultrasons (2) et $c_0$ représente la vitesse du son dans le fluide.

8. Système d'assistance selon la revendication 7, **caractérisé en ce que** l'équation diophantienne linéaire est résolue à l'aide de coefficients de Bézout ou d'une méthode d'exhaustion.

9. Système d'assistance selon la revendication 7 ou la revendication 8, **caractérisé en ce que** l'unité de traitement (3) est configurée pour fournir un écoulement de fluide calculé à partir de la vitesse d'écoulement.

# Fig. 1

# Fig. 2

1

2  15  18        13        16  17

3

# Fig. 3

a)        b)        c)

110       120       130

# Fig. 4

# Fig. 5

**IN DER BESCHREIBUNG AUFGEFÜHRTE DOKUMENTE**

**In der Beschreibung aufgeführte Patentdokumente**

- US 20080133006 A1

- US 20080210016 A1